# EUROPEAN PATENT SPECIFICATION

(11) **EP 0 805 201 B1**
(45) Date of publication and mention of the grant of the patent: **12.01.2000**
(21) Application number: 95925180.2
(22) Date of filing: 29.05.1995
(51) Int. Cl.: C12N 1/08

(54) **METHOD OF OBTAINING A BIOMASS OF MICROORGANISMS WITH A LOW NUCLEIC ACIDS CONTENT**
VERFAHREN ZUM ERHALT VON BIOMASSE AUS MIKROORGANISMEN MIT GERINGEM NUKLEINSÄUREGEHALT
PROCEDE D'OBTENTION D'UNE BIOMASSE DE MICRO-ORGANISMES AYANT UN FAIBLE CONTENU EN ACIDES NUCLEIQUES

(43) Date of publication of application: 05.11.1997
(73) Proprietor: Otkrytoe Akcionernoe Obschestvo"Nauchno-Issledovatelsky Institut Vychislitelnykh Komplexov im. M.A. Karceva",, Moscow, 117437 (RU); Orlova, Valentina Sergeevna, Moscow, 125475 (RU); Uvarov, Lev Alexeevich, Moscow, 117279 (RU); Evstigneev, Alexandr Alexandrovich, Moscow, 109029 (RU); Mukhtarulin, Valery Sergeevich, Moscow, 115470 (RU); Baranov, Lev Dmitrievich, Moscow, 117292 (RU); Orlova, Elena Vladimirovna, Moscow, 127273 (RU)
(72) Inventor: ORLOVA, Valentina Sergeevna, Moscow, 125475 (RU); UVAROV, Lev Alexeevich, Moscow, 117279 (RU); EVSTIGNEEV, Alexandr Alexandrovich, Moscow, 109029 (RU); MUKHTARULIN, Valery Sergeevich, Moscow, 115470 (RU); BARANOV, Lev Dmitrievich, Moscow, 117292 (RU); ORLOVA, Elena Vladimirovna, Moscow, 127273 (RU)
(74) Representative: Ebbinghaus, Dieter, Dipl.-Ing.
(86) International application number: RU9500105
(87) International publication number: WO9638535

(56) References cited:
- EP-A- 0 041 650
- EP-A- 0 050 831
- CH-A- 635 615
- DD-B- 207 849
- SU-A- 1 558 979
- US-A- 4 731 248
- TREVELYAN W E: "Chemical methods for the reduction of the purine content of baker's yeast, a form of single-cell protein" JOURNAL OF THE SCIENCE OF FOOD AND AGRICULTURE, vol. 27, 1976, pages 225-230, XP002070717
- OTERO M A ET AL: "RNA removal from Candida utilis by chemical and thermal treatments" ACTA BIOTECHNOLOGICA, vol. 2, no. 2, 1982, pages 145-153, XP002070718
- TREVELYAN W E: "Processing yeast to reduce its nucleic acid content. Induction of intracellular RNase action by a simple heat-shock procedure, and an efficient chemical method based on extraction of RNA by salt solutions at low pH" JOURNAL OF THE SCIENCE OF FOOD AND AGRICULTURE, vol. 29, 1978, pages 141-147, XP002070719

## Description

The invention relates to microbiology, namely to the technology of preparing a biomass of microorganisms with a low nucleic acid content and can be used in biotechnology plants for the preparation of protein-carbohydrate products and nucleic acid cleavage products.

Different processes for reducing the nucleic acid content in a biomass of microorganisms by cleavage with nucleases are known (A.A. Baskakova, S.I. Besborodova, M.I. Belyaeva and others, "Nuclease of microorganisms", M., Science, 1974, pp. 188-249, 277-279), some of these methods can be used in the microbiology industry.

A process for preparing a biomass of microorganisms with a low nucleic acid content by means of intercellular nucleases is also known (DE-2208279). According to this process the cells of microorganisms are washed with water, concentrated up to 5-15 mass-% by centrifuging, then airated at a temperature of 30°C. Afterwards, the pH is adjusted to 4,0 by means of a 5%-solution of NH₄OH, the biomass is maintained in this condition up to the beginning of the rise of the pH without adding NH₄OH, then the biomass is heated up to a temperature of 50-55°C, aeration is continued, the molarity is maintained up to 0,05-1,0 M by means of a buffer solution or by means of EDTA up to 0,01 M, then the pH value is increased up to 5,0-5,5 by adding HCl or CH₃COOH, and said conditions are maintained during 1-3 hours. Subsequently, the biomass is separated by centrifuging and dried.

Said process for preparing a biomass of microorganisms is a multistage one. For realization of this process the expensive reagent EDTA is required, which excludes its wide use in industry. Besides, the temperature during the process of reducing the nucleic acid content cannot be increased above 55°C; consequently, there is a loss of protein because of protease activity.

A process for preparing a protein product with a low nucleic acid content is known (SU-771154). This process comprises the dispersion of yeast biomass in a sodium chloride solution with subsequent heating, whereby the biomass is dispersed in a 1,5-8,0% sodium chloride solution. The heating of the biomass is carried out at a temperature of 65-80°C during 4-8 hours. Said process leads to a minor reduction of the nucleic acid content in the product.

A process for preparing a food protein product with a low nucleic acid content from a biomass of microorganisms is known (SU-791257), which comprises the extraction of a biomass by a solvent and ammonium hydroxide with subsequent separation of the extract and its drying. The extraction of the biomass is carried out by means of a mixture of a solvent and ammonium hydroxide in the ratio 1:3:0,03-1:6:0,06 by using as solvent propanol, glycol or monoglycol ether. This process allows to reduce the content of lipides and nucleic acids in the product. However, the use of propanol, glycol or monoglycol aldehyde for the extraction of the biomass results in a rise in the production costs.

A process for the production of a protein with reduced nucleic acid content from microorganisms is known (EP-A-0 050 831). Said process offers two variants for preparing a protein with a low nucleic acid content from microorganisms. According to one of the variants, microorganisms are treated with an acid and after their separation from the acid solution are extracted by means of a strong base. According to the other variant, the acid solution, containing nucleic acids, is neutralized before it is introduced into a fermentation zone. Acid anions and metallic cations, which are used at the neutralization stage, are forming inorganic compounds, which are favourable for the growth medium of the microorganisms in the fermentation stage. The process reduces the nucleic acid content, but does not result in its release into the culture medium.

The closest prior art describes a process for preparing yeasts with a low nucleic acid content (SU-1558979), according to which an initial biomass is heated in a boiling water-bath during 15-30 minutes, then cooled down to 37-40°C, treated by a hydrolysing agent, i.e. a fermentation reagent from Actinomyces coelicolor VKPM S-464, and hydrolysed at pH 9,0-10,5 in the presence of 5x10⁻³ M - 1,0x10⁻² M magnesium chloride for 5 to 6 hours, then the biomass is separated from the liquid phase. Thus, the use of the two-ferment reagent - 5'-exonuclease and alkaline phosphatase for the cleavage of nucleic acids and, moreover, the long duration of hydrolysis from 5 to 6 hours results in a significant increase of process expenses.

The invention is based on the problem of reducing the nucleic acid content, preparing hydrolysis products without using expensive ferment reagents and reactives, and reducing the duration of the process.

The subject matter of the present invention is a method for the preparation of a biomass of microorganisms with a low nucleic acid content comprising the steps of heating an initial biomass at a temperature of 75 to 90°C for 5 to 30 minutes, subsequently cooling the biomass to a temperature of 30 to 35°C and incubating the biomass with hydrolysis of the nucleic acids at a temperature of 30 to 35°C for 90 to 120 minutes, reheating the biomass at a temperature of 75 to 85°C and a pH in the medium of 1,0 to 1,5 for 30 to 60 minutes to release the hydrolysis products from the cells, increasing the pH of the medium up to 4,0 to 5,0, separating the resulting protein-carbohydrate product from the liquid phase, and finally concentrating and drying the obtained product.

According to the present process, the heating of the biomass at a temperature of 75-90°C during 5-30 minutes prevents insignificant cleavage of the protein by proteases and provides the activation of latent nucleases. The cooling of the biomass down to 30-35°C with its subsequent incubation during 90-120 minutes creates favourable conditions for manifestation of activity of intercellular nucleases with the subsequent cleavage of nucleic acids to low-molecular compounds.

The reheating of the biomass up to a temperature of 75-85°C during 30-60 minutes at a pH of 1,0-1,5 promotes the release of nucleic acid cleavage products from cells into the cultivation liquid. Before the separation of the obtained protein-carbohydrate product and nucleic acid cleavage products, the pH value of the medium is increased up to 4,5-5,0 in order to increase the guarantee of obtaining the final product.

The complex of operations of the present process and process conditions is novel, not known from the background of the invention, and guarantees the solution of the indicated problem, as it allows to obtain products of increased biological quality with low nucleic acid content without using expensive reagents and reactives.

The significant reduction of the duration of the technological process, about 2,0-2,5 times in comparison with the closest prior art and the simplicity of the apparatus also result in reducing the production costs and provides the possibility of a wide industrial application.

In a preferred embodiment the process of growing a biomass of microorganisms, namely of yeasts of the class of Saccharomyces cerevisiae, is carried out on a synthetic medium comprising glucose. The suspension of the biomass of yeasts is prepared in tap drinking water with a concentration of 50 g dry biomass/litre. At first, the biomass is denatured in a denucleatic reactor by means of heating at a temperature of 80-85°C during 10-15 minutes in a neutral medium (pH 6-7). Then the biomass is cooled and incubated, with hydrolysis of the nucleic acids, maintaining it at a temperature of 30-35°C during 100-110 minutes.

The hydrolysis products forming as a result of incubation are released by means of reheating the biomass and acidifying up to a pH value of 1,0-1,5 and maintaining it at a temperature of 75-80°C during 40-50 minutes. Then the suspension is diluted up to a concentration of 10 g dry biomass/litre. Afterwards the pH of the medium is increased up to 4,0-4,5 and the yeast cells are separated from the culture liquid by centrifuging. After drying the final protein-carbohydrate product with a low nucleic acid content is obtained.

The present process is illustrated by the following example as well as the data indicated in Tables 1 and 2.

### Example 1

Cells of Methylosinus trisporium, which were grown on methane as carbon source, have a low nucleic acid content of 2,8%. After the standard treatment of cells: heating at a temperature of 90°C during 5 minutes, subsequent incubation at a temperature of 34°C during 2 hours and reheating at a temperature of 80°C during 30 minutes in a medium with a pH of 1,0 - its quantity is reduced to 0,3%.

In Table 1 the parameters of the process for preparing a biomass with a low nucleic acid content are presented. It is evident from Table 1 that the duration of the present process is 2,0-2,5 times shorter in comparison with that of the closest prior art.

In Table 2 the characteristics of the obtained products are presented. From Table 2 it is evident that the residual nucleic acid content according to the present process is 3 times lower in comparison with that of the closest prior art, which testifies the efficiency of the present process.

### Industrial application

The present process of cultivation of a biomass of microorganisms can be applied in microbiological industry, namely in the technology of cultivation of a biomass with a low nucleic acid content for use in biotechnological plants with the purpose of obtaining protein-carbohydrate products and nucleic acid cleavage products.

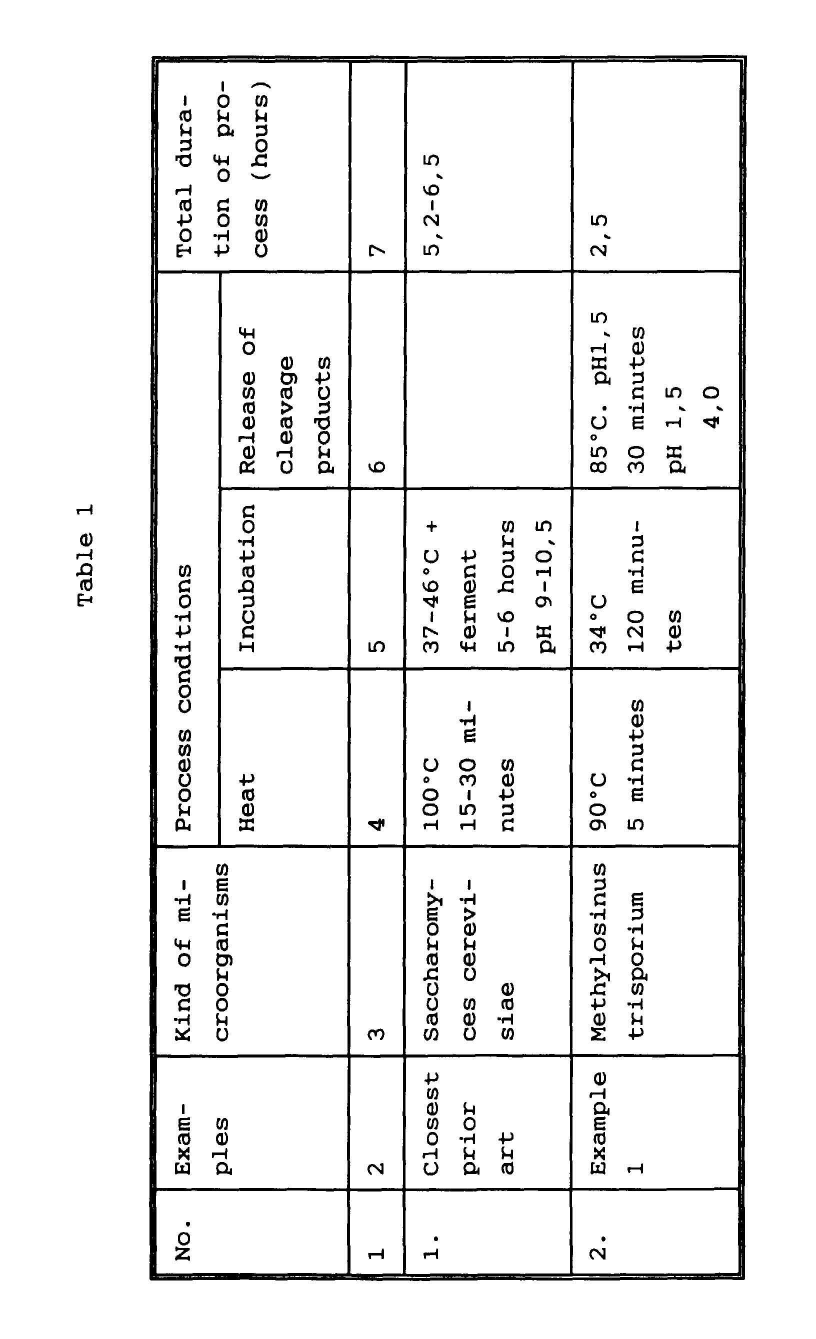

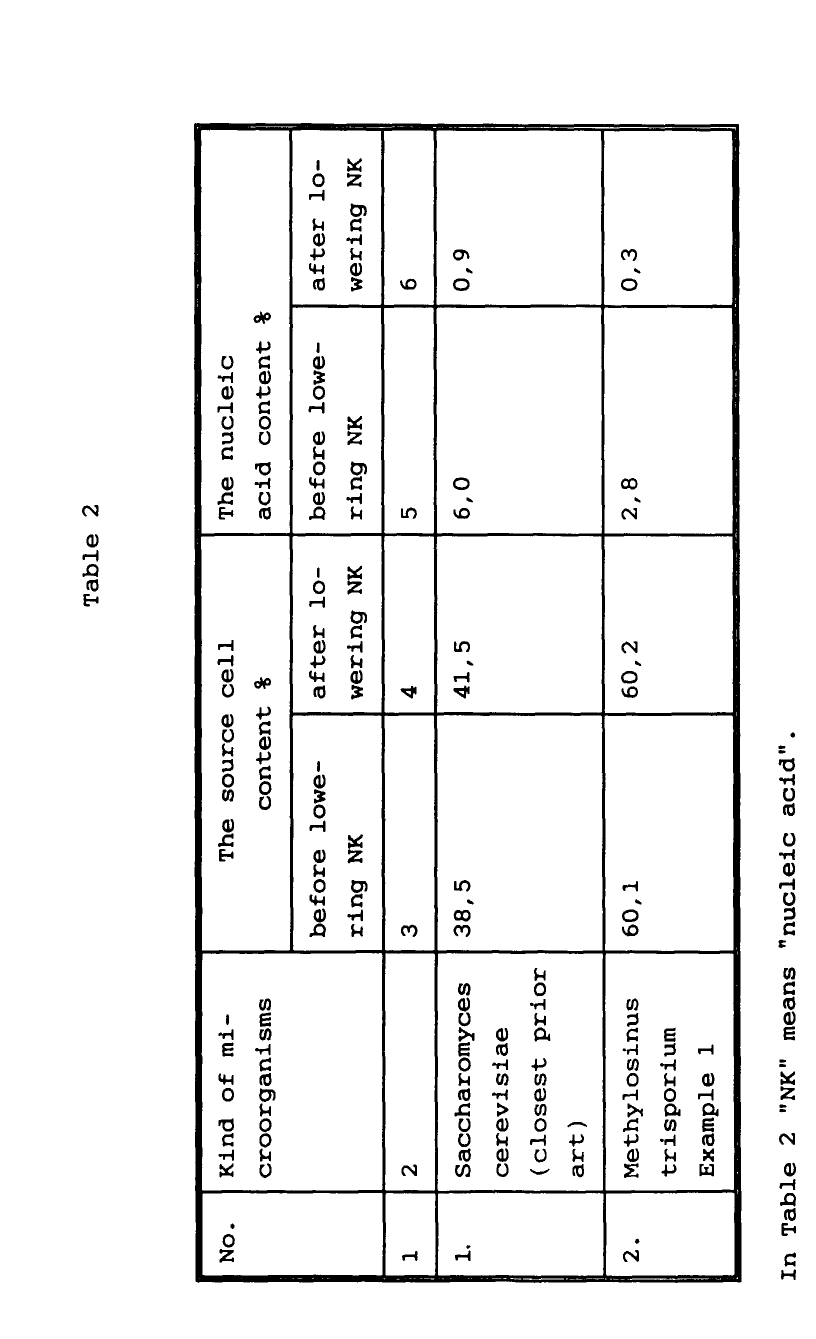

## Claims

1. A method for the preparation of a biomass of microorganisms with a low nucleic acid content comprising the steps of heating an initial biomass at a temperature of 75 to 90°C for 5 to 30 minutes, subsequently cooling the biomass to a temperature of 30 to 35°C, incubating the biomass with hydrolysis of the nucleic acids at a temperature of 30 to 35°C for 90 to 120 minutes, reheating the biomass at a temperature of 75 to 85°C and a pH in the medium of 1,0 to 1,5 for 30 to 60 minutes to release the hydrolysis products from the cells, increasing the pH of the medium up to 4,0 to 5,0, separating the resulting protein-carbohydrate product from the liquid phase, and finally concentrating and drying the obtained product.

## Patentansprüche

1. Verfahren zur Herstellung einer Biomasse von Mikroorganismen mit einem geringen Nucleinsäuregehalt, das die Stufen der Erwärmung der Ausgangsbiomasse bei einer Temperatur von 75 bis 90°C während 5 bis 30 Minuten, die nachfolgende Abkühlung der Biomasse auf eine Temperatur von 30 bis 35°C, die Inkubierung der Biomasse unter Hydrolyse der Nucleinsäuren bei einer Temperatur von 30 bis 35°C während 90 bis 120 Minuten, die erneute Erwärmung der Biomasse bei einer Temperatur von 75 bis 85°C und einem pH des Mediums von 1,0 bis 1,5 während 30 bis 60 Minuten zur Freisetzung der Hydrolyseprodukte aus den Zellen, die Steigerung des pH des Mediums auf 4,0 bis 5,0, die Abtrennung des erhaltenen Protein-Kohlehydrat-Produktes aus der flüssigen Phase und schließlich die Einengung und Trocknung des erhaltenen Produktes umfasst.

## Revendications

1. Méthode de préparation d'une biomasse de micro-organismes avec un faible contenu en acides nucléiques, comprenant les étapes de chauffage d'une biomasse initiale à une température de 75 à 90°C pendant 5 à 30 minutes, de refroidissement subséquent de la biomasse à une température de 30 à 35 °C, d'incubation de la biomasse par hydrolyse des acides nucléiques à une température de 30 à 35 °C pendant 90 à 120 minutes, de réchauffage de la biomasse à une température de 75 à 85 °C et à un pH dans le milieu de 1,0 à 1,5 pendant 30 à 60 minutes de façon à libérer les produits d'hydrolyse des cellules, d'accroissement du pH du milieu à 4,0 à 5,0, de séparation du produit protéine-carbohydrate résultant de la phase liquide et finalement de concentration et de séchage du produit obtenu.
